# EUROPEAN PATENT APPLICATION

(11) **EP 2 930 163 A1**
(43) Date of publication of application: **14.10.2015**
(21) Application number: 15162537.3
(22) Date of filing: 07.04.2015
(51) Int. Cl.: C07C 51/41, C07C 59/265, C08J 7/06

(54) **METHODS FOR CONVERTING MANGANESE DIOXIDE INTO WATER-SOLUBLE MANGANESE SALTS**

(30) Priority: 07.04.2014 US 201414246829
(71) Applicant: Baker Hughes Incorporated, Houston, TX 77210-4740 (US)
(72) Inventor: Hoover, Angela L., Tomball, Texas 77375 (US); Casey, Gregory E., Humble, Texas 77346 (US); Barden, Andrew J., Bellaire, Texas 77401 (US)
(74) Representative: Beacham, Annabel Rose

(57) **Abstract**

A water-insoluble manganese dioxide may be contacted with a solution having an effective amount of citric acid to form at least a portion of water-soluble manganese salt. The solution may have an initial pH of less than about 4. In a non-limiting embodiment, the solution may be fed into at least one equipment where the equipment has water-insoluble manganese dioxide therein.

## Description

### TECHNICAL FIELD

The present invention relates to methods for producing a water-soluble salt from manganese dioxide by contacting a water-insoluble manganese dioxide with a solution comprising an effective amount of citric acid.

### BACKGROUND

The presence of sulfur species in hydrocarbon fluids and aqueous streams is undesirable for various reasons. Non-limiting examples of sulfur species may be or include hydrogen sulfides, iron sulfides, calcium sulfides, and the like. Subterranean reservoirs currently being developed have increased amounts of sulfur species within the produced hydrocarbon streams (oil and gas). Hydrogen sulfide is a toxic gas that is heavier than air and is very corrosive to well and surface equipment.

During combustion, sulfur-rich hydrocarbon streams also produce heavy environmental pollution. When sulfur-rich streams contact metals, sulfur species lead to brittleness in carbon steels and to stress corrosion cracking in more highly alloyed materials. Moreover, sulfides and mercaptans, in various hydrocarbon or aqueous streams pose a safety hazard and a corrosion hazard. Removing these odorous and environmental malicious species would be desirable in oilfield, petrochemical, and refinery operations.

After inactivating as much of the sulfides from the hydrocarbon fluid as possible, the sulfides may be left behind as a precipitate and/or may react with the downstream equipment, such as refinery equipment. One method of inactivating (e.g. oxidizing) the sulfides from the downstream equipment involves chemically converting the sulfides to a different species by reacting the sulfides with a permanganate salt. 'Inactivate' with regards to the sulfides or other chemical species is defined herein to mean that the sulfides or other chemical species no longer react with a different chemical species within the hydrocarbon fluid and/or no longer react with equipment surfaces.

Non-limiting examples of the sulfides (one type of sulfur species) may be or include iron sulfides, calcium sulfides, hydrogen sulfides, and combinations thereof. Examples of the permanganate salt may be or include, but are not limited to, potassium permanganate, sodium permanganate, lithium permanganate. A non-limiting example of the reaction may be the following:

3H₂S+2KMnO₄→3S+2MnO₂+2H₂O+2KOH

As noted by the reaction above, such a reaction produces manganese (IV) dioxide (MnO₂). Manganese (IV) dioxide (MnO₂) is a strong oxidizer and is insoluble in water, nitric acid, or cold sulfuric acid, and it may slowly dissolve in aqueous HCl to give off Cl₂ gas. The manganese dioxide may also be left behind as a precipitate form within the downstream equipment. A standard removal of the manganese (IV) dioxide involves contacting the manganese dioxide with an oxalic acid and/or hydrochloric acid solution. However, the manganese (IV) dioxide and oxalic acid produce a manganese oxalate hydrate precipitate and/or manganese (II) salts, carbon dioxide gas, etc. within the downstream equipment. Moreover, oxalic acid and hydrochloric acid are difficult to handle for hazardous and toxicity reasons, and hydrochloric acid is highly corrosive and may produce hazardous chlorine gas.

It would be desirable if better methods were developed where less hazardous and/or corrosive acids may be used for reducing the manganese (IV) dioxide into water-soluble manganese salts.

### SUMMARY

There is provided, in one form, a method for producing a water-soluble species from manganese dioxide by contacting the water-insoluble manganese dioxide with a solution comprising citric acid. The solution may have an effective amount of citric acid present to form at least a portion of water-soluble manganese oxide. The solution may have an initial pH of less than about 4.

There is further provided in another non-limiting embodiment a method comprising feeding a solution into at least one downstream equipment where the downstream equipment may have water-insoluble manganese dioxide therein. The solution comprises citric acid, preferably in an amount ranging from about 1 wt% to about 5 wt% based on the total amount of the solution. The method may include reacting the water-insoluble manganese dioxide with the solution.

In yet another non-limiting embodiment, the method, described above, may further include removing the portion of the water-soluble manganese salt from the downstream equipment.

The citric acid is less hazardous for handling as compared to other acids that may be used for a similar purpose.

### DETAILED DESCRIPTION

It has been discovered that a water-soluble manganese salt may be produced by reacting a water-insoluble manganese dioxide with a solution comprising an effective amount of citric acid to produce the water-soluble manganese salt. The use of citric acid may reduce the water-insoluble manganese dioxide into water-soluble salts. The citric acid is less toxic, less hazardous, and less corrosive as compared to an otherwise identical method using a solution having oxalic acid and/or hydrochloric acid instead of the citric acid. Citric acid is a less hazardous acid than oxalic acid and/or hydrochloric acid, and the solution may be used during the final stage of removing water-insoluble manganese dioxide from the equipment, such as downstream equipment in one non-limiting embodiment. 'Downstream equipment' is defined herein as equipment downstream from a wellhead.

Converting all of the insoluble manganese dioxide into water-soluble manganese salt is a desirable goal, but it should be appreciated that complete conversion is not necessary for the methods discussed herein to be considered effective. Success is obtained if more of the water-insoluble manganese dioxide is converted (or reduced) to a water-soluble manganese salt using the solution comprising the citric acid than by using the solution absent the citric acid. Similarly, success is obtained if more of the water-soluble manganese salt is formed using the solution comprising the citric acid than by using the solution absent the citric acid. 'Effective amount' is defined herein to mean any amount of the citric acid that reduces more of the water-insoluble manganese dioxide into water-soluble manganese salts than in the absence of the citric acid.

'Dissolution' or 'dissolve' or 'soluble' with regards to the manganese salt is defined herein to mean that the manganese salt becomes present as a solute in a solution as a product of the reaction, i.e. the manganese salt may not precipitate out of solution. Said differently, the ions of the manganese salt are present in the solution as separate ions.

In a non-limiting example, the manganese of the water-insoluble manganese dioxide may be manganese (IV), and the manganese of the water-soluble manganese salt may be Mn(II). Preferably, the molar ratio of the water-insoluble manganese dioxide to the citric acid ranges from about 1:1 independently to about 1:10, alternatively from about 1:2 independently to about 1:8, or from about 1:4 independently to about 1:6. As used herein with respect to a range, "independently" means that any threshold may be used together with another threshold to give a suitable alternative range, e.g. about 1:8 to about 1:10 is a suitable alternative range.

Preferably, the methods may occur in a substantially oxygen-free environment, such as but not limited to the reaction between the water-insoluble manganese dioxide and the solution may occur in a substantially oxygen-free environment. 'Substantially-free' is defined herein to be an environment that is at least 60 vol% oxygen-free. Alternatively, the method(s) may occur in an environment ranging from about 60 vol% to about 100 vol% oxygen-free, or from about 90 vol% to about 99 vol% oxygen-free.

The solution may be fed into at least one downstream equipment (e.g. by using a valve and a hose attached to the downstream equipment) having a portion of water-insoluble manganese dioxide therein. Non-limiting examples of the downstream equipment may be or include, but are not limited to, heat exchangers, distillation towers, vessels, piping, fired heater tubes, and combinations thereof.

The water-insoluble manganese dioxide may react with the components of the solution and form a water-soluble manganese salt from the contacting/reaction between the water-insoluble manganese dioxide and citric acid in the solution. The water-soluble manganese salts may be removed (e.g. flushed) from the downstream equipment.

In another non-limiting embodiment, a permanganate salt may be fed into the downstream equipment prior to feeding the solution into the downstream equipment. The permanganate salt may react with at least one sulfur species present in the downstream equipment and form the water-insoluble manganese dioxide. The permanganate salt may be or include, but is not limited to, KMnO₄, NaMnO₄, and combinations thereof.

The effective amount of the citric acid within the solution is difficult to predict in advance because it would depend on the amount of the manganese dioxide present, the operating conditions (e.g. temperature), and the like. However, the solution may include the citric acid in an amount ranging from about 1 wt% independently to about 5 wt% based on the total amount of the solution, alternatively from about 2 wt% independently to about 3 wt%.

The solution may have optional components that aid the formation of the water-soluble manganese salt, such as but not limited to, catalysts, chelants, and combinations thereof. Reducing agents for manganese dioxide, such as but not limited to, sulfuric acid, oxalic acid, and/or hydrogen chloride may be present in the solution, but such reducing agents may decrease the ability of the water-insoluble manganese dioxide to be reduced into water-soluble manganese salts in the presence of citric acid. Preferably, the solution does not include sulfuric acid, oxalic acid, hydrogen chloride, or combinations thereof; stated differently, in one non-limiting embodiment, the method described herein is practiced in an absence of one or more of these chemicals.

Similarly, when the solution is fed into downstream equipment, the solution may react with other chemicals present within the downstream equipment, and this may cause a fluctuation in operating conditions (i.e. pH, temperature, pressure, etc.). It is difficult to predict specific operating conditions in advance for this reason, as well as whether the initial operating conditions may be maintained during the reaction between the solution and the water-insoluble manganese dioxide. Citric acid may target the water-insoluble manganese dioxide and other organic material present within the downstream equipment. The reaction(s) with the citric acid may form organic acid(s), in addition to the water-soluble manganese salt, and the formation of organic acid(s) may cause the final pH to drop. The final temperature and pressure may also vary depending on the reactions between the citric acid and other chemicals, besides the targeted water-insoluble manganese dioxide, within the downstream equipment.

However, the solution may have an initial pH less than about 4, alternatively from about 1.5 independently to about 3, alternatively from about 1.7 independently to about 2. A pH lower than about 1.5 may accelerate the reaction and/or induce corrosion to any downstream equipment. 'Initial pH' is defined herein as the pH of the solution at the time of mixing the components together but prior to contacting and/or reacting the solution with the water-insoluble manganese dioxide. Once the solution starts to react with the water-insoluble manganese dioxide, the final pH (i.e. the pH that occurs after all of the reactants have been reacted) may not be buffered to keep the pH the same as the initial pH; however, in one non-limiting instance, the initial pH may be maintained during the entirety of the contact and/or the reaction of the solution with the water-insoluble manganese dioxide.

The conditions during the contact and/or reaction between the water-insoluble manganese dioxide and the solution comprising citric acid may allow more of the water-soluble manganese salt to form at equilibrium favoring a forward reaction instead of a reversal of the reaction. The temperature for such a reaction may range from about 55 °C independently to about 75 °C, alternatively from about 60 °C independently to about 70 °C. The upper limits of the temperature may exceed 75°C, but it depends on the type of equipment involved. In the non-limiting instance of downstream equipment, the reaction may produce corrosion to the downstream equipment if the temperature exceeds about 75 °C. If the reaction goes below about 55 °C, the reaction may experience kinetic problems, and the products of the reaction may not form as fast and/or as well as they would if the temperature were above 55 °C.

The amount of time needed for such a reaction to occur may range from about 2 hours independently to about 12 hours, alternatively from about 4 hours independently to about 8 hours. The pressure for such a reaction may range from about atmospheric pressure to about 34.47 kPag (about 5 psig); however, in the instance of using the reaction to remove water-insoluble manganese dioxide from the downstream equipment, the pressure is not to exceed the limits of the downstream equipment.

The invention will be further described with respect to the following Examples, which are not meant to limit the invention, but rather to further illustrate the various embodiments.

### EXAMPLES

The ability and efficiency of solutions having oxalic acid, citric acid, and a combination of oxalic acid and citric acid were used to measure the dissolution of dry manganese dioxide. The testing was set up and encased in a nitrogen box. A total volume of 300 mL of solution was used for each test. The acid (oxalic acid, citric acid, or the combination of the two) was added to the DI water. The solution was then brought to about 65 °C using a thermocouple controlled heating stir plate. After the solution reached about 65 °C, and the box was flooded with N₂, the peristaltic pump began to cascade and circulate the solution over the (dry) Mn⁴⁺O₂. The pH and electromotive force (EMF) were recorded every 2 minutes for a 6-hour test period. The EMF was measured using a platinum (Pt) working electrode and a saturated calomel reference electrode. The components of each solution are noted in TABLE 1.

**TABLE 1**

| Component | Solution 1 (Comparative) | Solution 2 | Solution 3 |
|---|---|---|---|
| DI Water | 300 mL | 300 mL | 300 mL |
| Oxalic Acid | 6 g (0.067 mol) | 4.5 g (0.05 mol) | none |
| Citric Acid | none | 1.5 g (0.008 mol) | 6 g (0.03 mol) |
| MnO₂ | 1 g (0.012 mol) | 1 g (0.012 mol) | 1 g (0.012 mol) |

### EXAMPLE 1

Solution 1, Solution 2, and Solution 3 were tested having a pH of 3.5 under atmospheric conditions. When each solution was exposed to air, the reaction of Mn⁴⁺O₂ → Mn³⁺OOH occurred, and then reverted back to Mn⁴⁺O₂ due to the reduction by the oxygen in the air. This reaction prevented the Mn⁴⁺O₂ from being completely reduced to Mn²⁺O by oxalic acid, alone or in combination with citric acid.
Mechanism of Oxygen Reduction:

Mn⁴⁺O₂ + H₂O + e⁻ → Mn³⁺OOH + OH- (fast)

2 Mn³⁺OOH + O₂ → 2(Mn⁴⁺OOH* O⁻) (fast)

(Mn⁴⁺OOH * O⁻) + e⁻ → Mn⁴⁺O₂ + OH (slow)

### EXAMPLE 2

Solution 1, Solution 2, and Solution 3 having an initial pH of 3.5 were tested in an oxygen-free environment, and the pH was buffered at 3.5 during the entirety of the testing. Even in the oxygen-free environment, at pH 3.5, the three solutions were unable to completely dissolve the Mn⁴⁺O₂ into water-soluble manganese salts.

### EXAMPLE 3

Each solution was tested having an initial pH of 3.5; the final pH after testing was 1.8, and the MnO₂ was completely dissolved into water-soluble manganese salts within all of the solutions (oxalic acid, oxalic acid/citric acid combinations, and citric acid); said differently, 'completely dissolve' is defined to mean 100% of the manganese dioxide was reduced into water-soluble manganese salts. Solution 3 completely reduced the manganese dioxide to manganese salt after 4 hours. However, solution 1 reacted with MnO₂ formed a white powder precipitate, which was also formed from the reaction with solution 2 and MnO₂. The white powder precipitate was submitted for analytical analysis and confirmed to be a manganese oxalate hydrate precipitate, as noted by the reaction below.
Reaction of Oxalic Acid and MnO₂

2 C₂H₂O₄ (oxalic acid) + Mn⁴⁺O₂ → Mn²⁺C₂O₄·2H₂O + 2 CO₂

C₆H₈O₇ (citric acid) + Mn⁴⁺O₂ + 2H⁺ →C₅H₄O₅ + HCO⁻₃ + Mn²⁺O + 2 H₂O

In the foregoing specification, the invention has been described with reference to specific embodiments thereof, and has been described as effective in providing methods and compositions for producing a water-soluble manganese salt. However, it will be evident that various modifications and changes can be made thereto without departing from the broader spirit or scope of the invention as set forth in the appended claims. Accordingly, the specification is to be regarded in an illustrative rather than a restrictive sense. For example, specific water-insoluble manganese dioxides, water-soluble manganese salts, equipment, and the like falling within the claimed parameters, but not specifically identified or tried in a particular composition or method, are expected to be within the scope of this invention.

The present invention may suitably comprise, consist or consist essentially of the elements disclosed and may be practiced in the absence of an element not disclosed. For instance, the method for producing a water-soluble manganese salt may consist of or consist essentially of contacting the water-insoluble manganese dioxide with a solution having citric acid where the solution comprises an effective amount of citric acid to form at least a portion of water-soluble manganese salt, and the solution has an initial pH of less than about 4.

The words "comprising" and "comprises" as used throughout the claims, are to be interpreted to mean "including but not limited to" and "includes but not limited to", respectively.

## Claims

1. A method for producing a water-soluble manganese salt comprising:
contacting a water-insoluble manganese dioxide with a solution comprising an effective amount of citric acid to form at least a portion of water-soluble manganese salt; and wherein the solution has an initial pH of less than about 4.

2. The method of claim 1, wherein a reaction occurs between the water-insoluble manganese dioxide and the solution, and the method further comprises removing the water-soluble manganese salt from the reaction.

3. The method of claim 1 or claim 2 further comprising reacting a sulfur species with a permanganate salt to produce the water-insoluble manganese dioxide prior to contacting the water-insoluble manganese dioxide with the solution.

4. A method comprising:
feeding a solution into at least one downstream equipment having water-insoluble manganese dioxide therein; wherein the solution comprises citric acid in an amount ranging from about 1 wt% to about 5 wt% based on the total amount of the solution; and
reacting the water-insoluble manganese dioxide with the solution.

5. The method of claim 4, further comprising forming at least a portion of water-soluble manganese salt from the contacting of the water-insoluble manganese dioxide with the solution.

6. A method comprising:
feeding a solution comprising citric acid into at least one downstream equipment; wherein the at least one downstream equipment comprises water-insoluble manganese dioxide therein;
reacting the water-insoluble manganese dioxide with the solution to form at least a portion of a water-soluble manganese salt; and
removing the at least a portion of the water-soluble manganese salt from the at least one downstream equipment.

7. The method of any of claims 4 to 6, further comprising feeding a permanganate salt into the at least one downstream equipment prior to feeding the solution; wherein the permanganate salt reacts with at least one sulfur species present in the at least one downstream equipment to form the water-insoluble manganese dioxide.

8. The method of any of claims 4 to 7, wherein the solution has an initial pH of less than about 4.

9. The method of any preceding claim, wherein citric acid is present in the solution in an amount ranging from about 1 wt% to about 5 wt% based on the total amount of the solution.

10. The method of any preceding claim, wherein the method is carried out in a substantially oxygen-free environment.

11. The method of any preceding claim, wherein the molar ratio of the water-insoluble manganese dioxide to the citric acid ranges from about 1:1 to about 1:10.

12. The method of any preceding claim, wherein the solution does not comprise sulfuric acid, oxalic acid, hydrogen chloride, or combinations thereof.
